# EUROPÄISCHE PATENTSCHRIFT

(11) **EP 0 961 531 B1**
(45) Veröffentlichungstag und Bekanntmachung des Hinweises auf die Patenterteilung: **25.03.2009**
(21) Anmeldenummer: 99109069.7
(22) Anmeldetag: 07.05.1999
(51) Int. Cl.: H05K 1/18, A61N 1/02

(54) **Hochintegrierte elektronische Schaltung, insbesondere zum Einsatz in Herzschrittmachern**
Highly integrated electronic circuit, especially for using in heart stimulators
Circuit électronique à haute densité d'intégration, notamment pour l'utilisation dans des stimulateurs cardiaques

(30) Priorität: 15.05.1998 DE 19821857
(43) Veröffentlichungstag der Anmeldung: 01.12.1999
(73) Patentinhaber: BIOTRONIK GmbH & Co. KG, 12359 Berlin (DE)
(72) Erfinder: Schaldach, Max Jr., 14193 Berlin (DE)
(74) Vertreter: Lindner-Vogt, Karin L.

(56) Entgegenhaltungen:
- EP-A- 0 443 717
- CH-A- 677 054
- DE-A- 1 591 513
- DE-A- 4 123 407
- DE-U- 1 916 876
- US-A- 5 103 375
- US-A- 5 224 023
- US-A- 5 499 161
- US-A- 5 635 767
- US-A- 5 749 910

## Beschreibung

Die Erfindung betrifft eine hochintegrierte elektronische Schaltung, insbesondere zum Einsatz in Herzschrittmachern, Defibrillatoren und dergleichen, mit einem flexiblen Substrat, das eine Bestückungsseite und eine isolierende Rückseite aufweist, und mit einer der Schaltungsfunktion entsprechenden Anordnung von aktiven und passiven elektronischen Bauelementen auf der Bestückungsseite des Substrates, die auf Kontaktierflächen des Substrates befestigt und durch Leiterbahnen auf dem Substrat entsprechend der Schaltungsfunktion verbunden sind.

Zum Hintergrund der Erfindung ist festzuhalten, daß bei hochintegrierten elektronischen Schaltungen Komponenten unterschiedlicher Art, also aktive und passive elektronische Bauelemente, elektrisch miteinander verbunden werden. Das Substrat besteht dabei aus einem elektrisch isolierenden, dielektrischen Material, auf dem Kontaktflächen zur Kontaktierung der Bauelemente und elektrische Leiterbahnen zu deren Verbindung untereinander vorhanden sind. Weitere Kontaktflächen können als Schnittstellen zur Anbindung der hochintegrierten elektronischen Schaltung an externe Komponenten, wie eine Stromversorgung, Telemetrie-Komponenten und dergleichen vorgesehen sein. Damit wird die hochintegrierte elektronische Schaltung in das Umfeld eines bestimmten Gerätes eingebunden. Die Kontaktierung der Kontaktflächen erfolgt z.B. durch Löten.

Insbesondere beim Einsatz in Herzschrittmachern, Defibrillatoren und ähnlichen Geräten spielt die Miniaturisierung von solchen hochintegrierten elektronischen Schaltungen eine herausragende Rolle. Mit größerer Kompaktheit der Schaltung kann die Gesamtgröße von solchen implantierbaren medizinischen Geräten reduziert werden, was aus medizinischer Sicht zur Vereinfachung der notwendigen operativen Eingriffe und zu besseren Trageeigenschaften für den Patienten führt.

Grundsätzlich ist ferner die Minimierung der Anzahl unterschiedlicher Herstellungsschritte zur Fertigungsrationalisierung und zur gleichzeitigen Erhöhung der bei solchen Geräten unabdingbaren Zuverlässigkeit ein anzustrebendes Ziel.

Zum Stand der Technik ist festzuhalten, daß herkömmliche hochintegrierte elektronische Schaltungen auf der Basis starrer keramischer Substrate aufgebaut sind. Zur Erhöhung der Packungsdichte der Bauelemente kann dabei deren Größe selbst reduziert und die Kontaktierung möglichst platzsparend ausgeführt werden. In diesem Zusammenhang ist beispielsweise die SMD-(= Surface-Mounted-Device-) Technologie zu nennen, bei der die oberflächenkontaktierten Bauelemente keine Anschlußbeine mehr aufweisen. Auch die Verwendung mehrerer Leiterbahn-Ebenen übereinander in einem Substrat ist als-Maßnahme zur Erhöhung der Packungsdichte bekannt.

Ferner gibt der Stand der Technik bereits Schaltungen an, die auf flexiblen Kunststoff-Substraten aufgebaut sind. Mittels Lithographie-Verfahren können auf solchen flexiblen Substraten sehr schmale Leiterbahn-Strukturen mit geringen Abständen aufgebracht werden, was erheblich zur Miniaturisierung der Schaltung beitragen Kann.

Explizit zeigt dabei die US-A-5386341 die Verwendung eines U-förmig zusammengefalteten flexiblen Substrates, wobei zwischen den gefalteten Schenkeln des Substrates eine Versteifungsplatte eingesetzt ist. Auf der Oberseite dieser Sandwich-Struktur sitzen Halbleiter-Bauelemente, während die Unterseite über Lötpunkte mit einer übergeordneten Leiterplatte verbunden ist. Durch das flexible Substrat werden unterschiedliche thermische Ausdehnungskoeffizienten von Leiterplatte und elektronischen Bauelementen ausgeglichen.

Aus der US-A-5362656 ist es ferner bekannt, um ein starres Metallsubstrat ein flexibles Leiterbahnteil herumzuwickeln, wobei die Leiter auf dem Leiterbahnteil mit einem integrierten Schaltkreis verbunden werden, der auf der Oberfläche des Substrats montiert ist. Das flexible Leiterbahnteil hat eine Vielzahl von metallischen Anschlußflächen, die der Unterseite des Substrates benachbart angeordnet sind. Über Signalleitungen im flexiblen Leiterbahnteil wird der integrierte Schaltkreis mit den metallischen Anschlußflächen gekoppelt. Der Anschlußflächen wiederum können an eine gedruckte Schaltplatine angelötet werden, um den integrierten Schaltkreis elektrisch mit der Platine zu koppeln.

Aus der DE-A-4 123 407, EP-A-0 443 717, US-A-5 103 375, DE-A-1 591 513 und DE-U-1 916 876 sind Schaltungsanordnungen bekannt, bei denen das Substrat entlang eines Faltbereiches derart zusammengefaltet ist, daß die beiderseits des Faltbereiches liegenden, jeweils mit Bauelementen bestückten Abschnitte des Substrates etwa parallel und eng aneinander angeordnet sind. Die hochintegrierte elektronische Schaltung ist einerseits durch die Faltung und die Verwendung eines in der Regel sehr dünnen flexiblen Substrates sehr kompakt. Das Zusammenfalten findet dabei ohne mechanisch stützende Zwischenlage statt, was der Kompaktheit der Schaltung zugute kommt. Die beispielsweise in der DE-A-4 123 407 gezeigte nur einseitige Anwendung von elektronischen Bauelementen auf der Bestückungsseite des Substrates erlaubt die Verwendung lediglich eines einzigen Kontaktier-Verfahrens in einem einzigen Herstellungsschritt.

Bei diesen gezeigten Schaltungsanordnungen sind - wie beispielsweise aus der US-A 5,103,375 erkennbar ist - gesonderte Anschlüsse in Form von Stecker- oder Buchsenleisten vorgesehen oder es müssen entsprechende Anschlussleitungen auf Lötflächen des Substrates angelötet werden, was beschädigungsempfindliche Kontaktstellen für den Anschluss der Schaltungsanordnung mit sich bringt.

Der Erfindung liegt davon ausgehend die Aufgabe zu Grunde, eine elektronische Schaltung auf der Basis eines flexiblen Substrates so auszugestalten, dass deren Anschlüsse produktionstechnisch einfach und störunempfindlicher ausgestaltet sind.

Diese Aufgabe wird durch die im Kennzeichnungsteil des Anspruches 1 angegebenen Merkmale gelöst, wonach das Substrat mit einstückig angesetzten, flexiblen Anschlusslaschen zur Verbindung der Schaltung mit externen Komponenten versehen ist.

Die Unteransprüche betreffen vorteilhafte Weiterbildungen des Erfindungsgegenstandes, die in der nachfolgenden Beschreibung eines Ausführungsbeispiels der Erfindung anhand der beigefügten Zeichnungen näher erläutert werden. Es zeigen:
- Fig. 1: eine Draufsicht auf die Bestückungsseite eines flexiblen Substrates vor der Bestückung mit Bauelementen,
- Fig. 2: eine schematische Draufsicht auf ein Substrat in einer zweiten Ausführungsform,
- Fig. 3: eine schematische Seitenansicht einer hochintegrierten elektronischen Schaltung in bestücktem und zusammengefalteten Zustand des flexiblen Substrates,
- Fig. 4: eine schematische Detaildarstellung für die Anbringung aktiver Bauelemente am Substrat unter Zwischenschaltung von passiven Bauelementen und
- Fig. 5: eine noch detailliertere schematische Darstellung eines Lötpunktes an einem aktiven Bauelement.

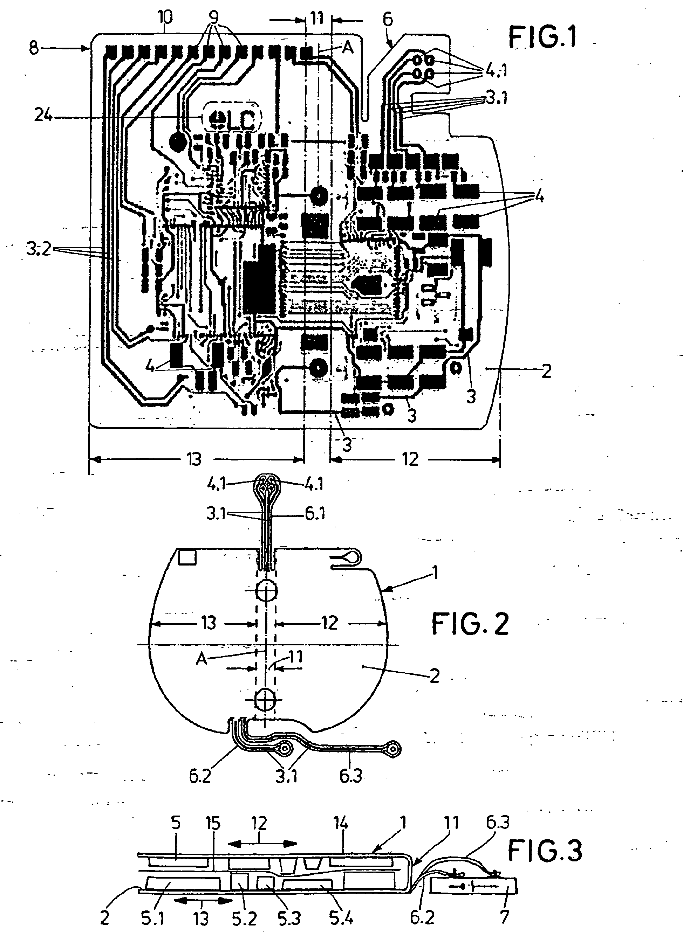

Wie aus Fig. 1 deutlich wird, weist ein aus flexibler Kunstoffolie bestehendes Substrat 1 auf seiner in der Zeichnung sichtbaren Bestückungsseite 2 eine der Schaltungsfunktion entsprechende Anordnung von lithographisch oder lasertechnisch (oder durch eine Kombination beider Verfahren) aufgebrachten metallischen Leiterbahnen 3 und Kontaktierflächen 4 auf. Letztere stellen sich durch in ihren Ausmaßen gegenüber den Leiterbahnen 3 vergrößerte Rechtecke, Quadrate, Kreise oder Kreisringe dar. Auf diesen Kontaktierflächen 4 werden in noch näher zu erläuternder Weise Bauelemente 5 aufgelötet, die damit entsprechend der Schaltungsfunktion über die Leiterbahnen 3 miteinander verbunden sind.

Wie aus Fig. 1 ferner deutlich wird, ist bei diesem Ausführungsbeispiel das Substrat 1 mit einer L-förmigen, einstückig angesetzten und damit ebenso wie das Substrat 1 flexiblen Anschlußlasche 6 versehen, auf der vier Leiterbahnen 3.1 jeweils zu einer Kontaktierfläche 4.1 führen, an denen eine elektrische vakuumdichte Durchführung zur Kontaktierung des Elektrodensteckers durch das vakuumdichte Schrittmachergehäuse hindurch angeschlossen werden kann. In Fig. 2 ist eine derartige Anschlußlasche 6.1 mit Leiterbahnen 3.1 und Kontaktierflächen 4.1 für den gleichen Zweck als geradlinig angesetztes Teil mit einem verbreiterten Ende dargestellt, auf dem gleichzeitig eine Kontaktierfläche für die Masseverbindung Gehäuse-Schaltung vorgesehen ist. An der gegenüberliegenden Seite sind weitere, langgestreckte Anschlußlaschen 6.2 und 6.3 einstückig angeformt, über die eine Batterie 7 (Fig. 3) zur Spannungsversorgung der hochintegrierten elektronischen Schaltung auf dem Substrat 1 angekoppelt werden kann.

In Fig. 1 ist ferner eine Reihe 8 von Test-Anschlußflächen 9 erkennbar, die an einem Rand 10 des Substrates 1 liegt. Die Testanschlußflächen 9 sind über verschiedene Leiterbahnen 3.2 zu verschiedenen Punkten der Schaltung durchgeschleift. Nach dem Bestücken des Substrates 1 kann die so geschaffene Schaltung über die Anschlußflächen 9 sehr schnell auf ihre Funktionsfähigkeit durch Einsatz eines mehrfingrigen Kontaktarmes getestet-werden. Danach kann der die Test-Anschlußflächen 9 und Leiterbahnen 3.2 tragende Bereich des Substrates 1 entfernt werden, so daß das Substrat eine um die Achse A gemäß Fig. 1 ungefähr symmetrische Kontur aufweist. Dies bedeutet, daß der linke Teil des in Fig. 1 gezeigten Substrates 1 genauso halbkreisförmig abschließt, wie dessen rechter Teil. Zu den Test- Anschlußflächen 9 ist zu ergänzen, daß diese einseitig aufgebracht und gemäß einer standardisierten Test-Kontakt-Struktur ausgebildet sind. Sie machen ein umständliches Anfahren einzelner Test-Kontakt-Punkte innerhalb der Schaltung überflüssig und erlauben die Standardisierung über unterschiedliche Produktlinien hinweg.

Um nun den Flächenbedarf der gesamten hochintegrierten elektronischen Schaltung trotz der nur einseitigen Bestückung auf der Bestückungsseite 2 und der damit grundsätzlich doppelt großen aktiven Fläche des Substrats auf das Maß einer beidseitig bestückten hochintegrierten elektronischen Schaltung zu reduzieren, wird - wie Fig. 3 erkennen läßt - das flexible Substrat um die Achse A, d.h. etwa mittig, zusammengeklappt, so daß die beiderseits des Faltbereiches 11 liegenden Abschnitte 12, 13 des Substrates mit ihren Bestückunpsseiten einander zugewandt sind. Die passive Rückseite 14 des Substrates 1 bildet die Isolierung der hochintegrierten elektronischen Schaltung gegenüber dem Gehäuse und anderen Komponenten des damit ausgerüsteten Herzschrittmachers. In der in Fig. 3 gezeigten, etwa U-förmigen Konfiguration der Schaltung liegen die jeweils mit nach innen liegenden Bauelementen 5, wie z.B. IC's 5.1, Dioden 5.2, Kondensatoren 5.3 oder Widerständen 5.4, bestückten Abschnitte 12,13 eng aneinander. Durch diese Anordnung wird eine integrierte Deckelung der Schaltung bewirkt. Um nun eine saubere elektrische Trennung zwischen den parallelen Abschnitten 12, 13 des Substrates 1 auf der Bestückungsseite 2 zu gewährleisten, ist dazwischen eine Isolierlage 15 z.B. in Form eines nichtleitenden Folienblattes eingelegt.

Wie in Fig. 3 nicht dargestellt ist, kann analog der Isolierlage 15 auch ein Bauteil des mit der hochintegrierten elektronischen Schaltung ausgerüsteten Herzschrittmachers zwischen die beiden Abschnitte 12, 13 des zusammengefalteten Substrates eingelegt werden. Dies kann beispielsweise die Telemetrie-Spule sein, die ansonsten als außerhalb der hochintegrierten elektronischen Schaltung liegendes Teil eines besonderen Handhabungsaufwandes bedarf. Bei der vorstehend erörterten Positionierung der Telemetrie-Spule werden deren Anschlußleitungen in der Nähe des Faltbereiches 11 an eine entsprechende Kontaktfläche - beispielsweise die Kontaktflächen 4.1 in Fig. 1 - angelötet und anschließend das bestückte Substrat unter Zwischenlage der Telemetrie-Spule zusammengeklappt. Für diesen Fall ist die Isolierlage 15 entbehrlich, da die Spule ohnehin isoliert und zusätzlich noch in eine Kunststoffolie eingebettet werden kann.

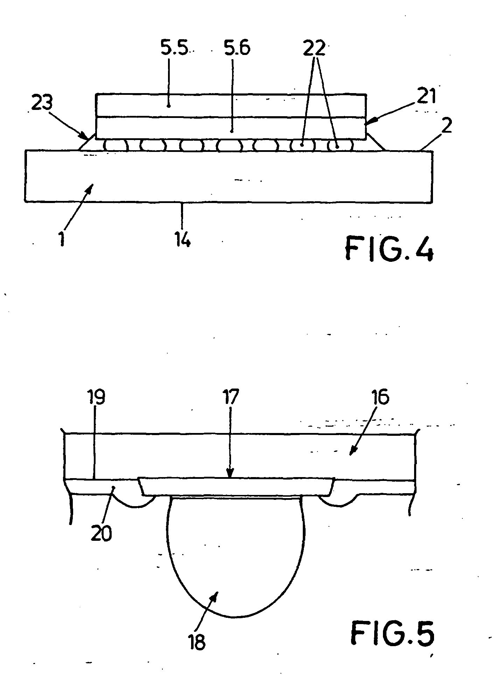

Wie in den Fig. 4 und 5 schematisch angedeutet ist, kann zur weiteren Reduzierung des Flächenbedarfs und der Anzahl der Herstellungsschritte für die hochintegrierte elektronische Schaltung der Einsatz von Halbleiter-Bauelementen 5.5 in der sogenannten Chip-Scale-Package-Technologie beitragen. Dabei werden grundsätzlich Halbleiter-Bauelemente nicht mehr mit der beispielsweise über Miniaturdrähte zu kontaktierenden, aktiven Seite vom Substrat abgewandt angebracht, sondern mit der aktiven Seite dem Substrat 1 zugewandt aufgesetzt. Die Befestigung und Kontaktierung erfolgt über Lot-Tropfen, wie dies in Fig. 5 dargestellt ist. So ist dort die aktive Silizium-Wafer-Lage 16 des Halbleiter-Chips mit einer Anschlußfläche 17 versehen, auf der ein Lot-Tropfen 18 aufgebracht ist. Die Unterseite 19 der Silizium-Wafer-Lage 16 und der Rand der Anschlußfläche 17 sind durch eine für die Lithographie erforderliche Benzo-Cyklo-Buten-Schicht 20 isoliert. Die beschriebenen Bauelemente 5 werden auf der in Fig. 5 gezeigten Weise mit Lot-Tropfen 18 an ihren Anschlußflächen 17 oder entsprechenden Kontaktflächen versehen und auf das aufgeklappte, ebene Substrat 1 gesetzt. Durch ein einmaliges Aufheizen der so vorkonfektonierten Anordnung erfolgt das Verlöten aller Bauelemente 5 mit einem einheitlichen Löt-Kontaktierverfahren in einem einzigen Herstellungsschritt. In diesem Zusammenhang ist darauf hinzuweisen, daß statt der Bauelemente 5 auch die Kontaktierflächen 4 auf dem Substrat 1 mit entsprechenden Lot-Tropfen versehen, anschließend die Bauelemente aufgesetzt und wiederum durch Temperatureinfluß ein gleichzeitiges Verlöten aller Bauelemente stattfinden kann. Stattdessen kann der Lötprozeß auch durch einen Klebe-Prozeß (zur Vermeidung des Blei im Lot) ersetzt werden.

Die vorstehend erörterte Chip-Scale-Package-Technologie hat im übrigen weiterhin den Vorteil, daß sie die Kompaktheit der Schaltung erhöht. Zum einen liegt dies daran, daß das Anbringen von Schutzgehäusen über den dabei verwendeten Halbleiter-Bauelementen verzichtbar ist, da vom Substrat 1 abgewandt und ungeschützt nicht die empfindliche aktive, sondern lediglich deren unempfindliche passive Seite offen liegt. Zum anderen eröffnet die Chip-Scale-Package-Technologie die Möglichkeit, ein Halbleiter-Bauelement 5.5, wie es z.B. in Fig. 4 gezeigt ist, und Substrat 1 über eine funktionale dazwischenliegende Umverdrahtungsschicht 21 mit einem darin integrierten passiven Bauelement, wie z.B. einem Widerstand 5.6, zu verbinden. Ein aktives Bauelement 5.5 kann also über die mit passiven Bauelementlagen versehene Umverdrahtungsschicht 21 auf dem Substrat 1 sitzen. Die Verbindung erfolgt dabei wiederum über Lötstellen, die im Fachjargon als "Flip-Chip-Bump" bezeichnet werden. Diese Lötstellen 22 sind zusammen mit der Unterseite des passiven Bauelementes 5.6 in eine Epoxidharzschicht 23 eingebettet.

Die vorstehend erwähnte Einbindung passiver Widerstände oder anderer passiver Bauelemente als Verbindungselemente ermöglicht deren Herstellung in einer beliebigen, von der Herstellung des Substrat unabhängigen Technologie und deren anschließende Anbindung in einem einzigen Herstellungsschritt. Zudem wird ein für abweichende hochintegrierte elektronische Schaltungen modularer Aufbau möglich, bei dem die Anbindung anderer Halbleiter-Bauelemente mit anderen Spezifikationen an die passiven Bauelemente lediglich den Einsatz eines anderen passiven Bauelementes in der Verbindung mit dem aktiven Bauelement, nicht aber ein anderes Leiterbahn-Design auf dem Substrat 1 erforderlich macht.

Der Verzicht auf mehrere Temperaturschritte beim Bestücken, die jeweils zu Änderungen der Substrat-Eigenschaften führen, so wie die höhere-Präzision der Leiterbahnen durch deren lithographische Strukturierung machen die parallele Herstellung mehrerer hochintegrierter elektronischer Schaltungen in Vielfach-Nutzen und anschließende Vereinzelung möglich. Herkömmliche starre Keramiksubstrate sind aufgrund der geringeren Präzision herkömmlicherweise nur in Einfach-Nutzen herzustellen.

Zum Substrat 1 ist noch zu erwähnen, daß dessen typische Dielektrikumslagen-Dicke im Bereich von etwa 50 µm liegt. Um ausreichende mechanische Stabilität zu erzielen, ist daher noch eine weitere Versteifungsschicht mit einer Dicke von etwa 400 µm vorgesehen, die außerdem die elektrische Isolierung der Rückseite 14 des Substrates 1 vornimmt. Das Material des Substrates ist in der Regel Poly-Imid.

Unter nochmaliger Bezugnahme auf Fig. 1 ist zu ergänzen, daß das Substrat 1 direkt mit einer röntgensichtbaren Markierung 24 - hier in Form eines Firmenlogos und der Buchstabenkombination "LC" - versehen ist. Damit ist eine Röntgenmarkierung direkt in das Substrat integriert. Bei den bisherigen starren hochintegrierten elektronischen Schaltungen war dies nicht möglich, da die Röntgenmarkierung einen wesentlichen Flächenbedarf aufweist, der bei der knappen verfügbaren Substrat-Oberfläche nicht akzeptiert wurde. Insoweit wurde bisher die Röntgenmarkierung in einem gesonderten Bearbeitungsschritt in eine andere Komponente z.B. eines Herzschrittmacher integriert.

## Patentansprüche

1. Hochintegrierte elektronische Schaltung zum Einsatz in Herzschrittmachern oder Defibrillatoren mit
- einem flexiblen Substrat (1), das eine Bestückungsseite (2) und eine isolierende Rückseite (14) aufweist, und
- einer der Schaltungsfunktion entsprechenden Anordnung von aktiven und passiven elektronischen Bauelementen (5) auf der Bestückungsseite (2) des Substrates (1), die auf Kontaktierflächen (4) des Substrates (1) befestigt und durch Leiterbahnen (3) auf dem Substrat (1) entsprechend der Schaltungsfunktion verbunden sind
**dadurch gekennzeichnet, daß**
- das Substrat (1) entlang eines Faltbereichs (11) derart zusammeragefaltet ist, daß die beiderseits des Faltbereiches (11) liegenden, jeweils mit Bauelementen (5) bestückten Abschnitte (12,13) des Substrates (1) etwa parallel und eng aneinander angeordnet sind.

2. Schaltung nach Anspruch 1, **dadurch gekennzeichnet, daß** der Faltbereich (11) etwa mittig bezogen auf die Gesamtfläche des Substrates (1) angeordnet ist, so daß die Schaltung eine etwa U-förmige Konfiguration aufweist.

3. Schaltung nach Anspruch 1 oder 2, **dadurch gekennzeichnet, daß** die Bestückungsseite (2) des Substrates (1) im gefalteten Zustand innen liegt.

4. Schaltung nach Anspruch 3, **dadurch gekennzeichnet, daß** zwischen den einander zugewandten Bauelementen (5) auf den jeweiligen Abschnitten (12,13) des Substrates (1) eine Isolierlage (15) gelegt ist.

5. Schaltung nach einem der Ansprüche 1 bis 4,**dadurch gekennzeichnet, daß** das Substrat (1) mit einstückig angesetzten, flexiblen Anschlußlaschen (6) zur Verbindung der Schaltung mit externen Komponenten (7) versehen ist.

6. Schaltung nach einem der Ansprüche 1 bis 5, **dadurch gekennzeichnet, daß** das Substrat (1) mit standardisierten Test-Anschlußflächen (9) versehen ist, die vor dem Zusammenfalten des bestückten Substrates (1) entfernbar sind.

7. Schaltung nach Anspruch 6, **dadurch gekennzeichnet, daß** die Test-Anschlußflächen (9) in einer am Rand (10) des Substrats (1) liegenden Reihe (8) angeordnet sind.

8. Schaltung nach einem der Ansprüche 1 bis 7, **dadurch gekennzeichnet, daß** eine röntgensichtbare Markierung (24) auf dem Substrat (1), vorzugsweise auf einer vom Rand des Substrates abstehenden, umfaltbaren Lasche angeordnet ist.

9. Schaltung nach einem der Ansprüche 1 bis 8, **dadurch gekennzeichnet, daß** alle Bauelemente (5) auf der Bestückungseite (2) mit einem einheitlichen Kontaktierverfahren in einem einzigen Herstellungsschritt aufmontiert, insbesondere aufgelötet oder geklebt sind.

10. Schaltung nach einem der Ansprüche 1 bis 9, **dadurch gekennzeichnet, daß** aktive Bauelemente (5.5) huckepack auf passiven Bauelementen (5.6) angeordnet sind, die als Verbindungselemente zwischen dem jeweiligen aktiven Bauelement (5.3) und dem Substrat (1) fungieren.

11. Schaltung nach einem der Ansprüche 1 bis 10, **dadurch gekennzeichnet, daß** externe Komponenten, insbesondere die Telemetrie-Spule eines Herzschrittmachers, Defibrillators oder dergleichen, zwischen den beiden bestückten Abschnitten (12, 13) des gefalteten Substrates (1) eingelegt sind.

12. Schaltung nach einem der Ansprüche 1 bis 11, **gekennzeichnet durch** eine lithographische Strukturierung der Leiterbahnen (3).

13. Schaltung nach einem der Ansprüche 1 bis 12, **gekennzeichnet durch** eine lasertechnische Strukturierung der Leiterbahnen (3).

## Claims

1. A highly integrated electronic circuit for use in pacemakers or defibrillators, having
- a flexible substrate (1), which has an assembly side (2) and an insulating reverse side (14), and
- an arrangement of active and passive electronic components (5) on the assembly side (2) of the substrate (1), corresponding to the circuit function, these components being attached to the contact faces (4) of the substrate (1) and connected by printed conductors (3) to the substrate (1) according to the circuit function,
**characterized in that**
- the substrate (1) is folded along a fold area (11), such that the sections (12, 13) of the substrate (1) situated on both sides of the fold area (11) and assembled with components (5) are arranged approximately in parallel and close to one another.

2. The circuit according to Claim 1, **characterized in that** the fold area (11) is arranged approximately at the center based on the total area of the substrate (1), so that the circuit has an approximately U-shaped configuration.

3. The circuit according to Claim 1 or 2, **characterized in that** the assembly side (2) of the substrate (1) is on the inside in the folded state.

4. The circuit according to Claim 3, **characterized in that** an insulating layer (15) is placed between the facing components (5) on the respective sections (12, 13) of the substrate (1).

5. The circuit according to any one of Claims 1 to 4, **characterized in that** the substrate (1) is provided with flexible connecting straps (6) attached in one piece for connecting the circuit to external components (7).

6. The circuit according to any one of Claims 1 to 5, **characterized in that** the substrate (1) is provided with standardized test terminal faces (9), which can be removed before folding together the assembled substrate (1).

7. The circuit according to Claim 6, **characterized in that** the test terminal faces (9) are arranged in a row (8) situated at the edge (10) of the substrate (1).

8. The circuit according to any one of Claims 1 to 7, **characterized in that** a marker (24) visible to X-rays is provided on the substrate (1), preferably on a strap that can be folded over and protrudes away from the edge of the substrate.

9. The circuit according to any one of Claims 1 to 8, **characterized in that** all the components (5) are mounted on the assembly side (2), in particular being soldered or adhesively bonded by a uniform contacting method in a single manufacturing step.

10. The circuit according to any one of Claims 1 to 9, **characterized in that** active components (5.5) are arranged in piggyback fashion on passive components (5.6) that function as connecting elements between the respective active component (5.3) and the substrate (1).

11. The circuit according to any one of Claims 1 to 10, **characterized in that** external components, in particular the telemetry coil of a pacemaker, defibrillator or the like, are inserted between the two assembled sections (12, 13) of the folded substrate (1).

12. The circuit according to any one of Claims 1 to 11, **characterized by** a lithographic structuring of the printed conductors (3).

13. The circuit according to any one of Claims 1 to 12, **characterized by** a laser structuring of the printed conductors (3).

## Revendications

1. Circuit électronique à haute intégration destiné à une utilisation des pacemakers ou des défibrillateurs, comprenant
- un substrat (1) flexible, qui présente un côté à équiper (2) et une face postérieure (14) isolante, et
- un agencement, correspondant à la fonction de commutation de composants (5) électroniques actifs et passifs sur le côté à équiper (2) du substrat (1), qui sont fixés sur des surfaces d'établissement de contact (4) du substrat (1) et sont reliés par des pistes conductrices (3) sur le substrat (1) conformément à la fonction de commutation,
**caractérisé en ce que**
- le substrat (1) est replié le long d'une zone de pliage (11) de telle sorte que les parties (12, 13), situées des deux côtés de la zone de pliage (11) et équipées chacune de composants (5), du substrat (1) sont disposées de façon à peu près parallèle et très près les unes des autres.

2. Circuit selon la revendication 1, **caractérisé en ce que** la zone de pliage (11) est disposée à peu près au centre par rapport à la surface globale du substrat (1), de sorte que le circuit présente une configuration à peu près en U.

3. Circuit selon la revendication 1 ou 2, **caractérisé en ce que** le côté à équiper (2) du substrat (1) est situé à l'intérieur dans l'état plié.

4. Circuit selon la revendication 3, **caractérisé en ce qu'**une couche isolante (15) est située entre les composants (5), tournés les uns vers les autres, sur les parties (12, 13) respectives du substrat (1).

5. Circuit selon l'une quelconque des revendications 1 à 4, **caractérisé en ce que** le substrat (1) est doté de pattes de raccordement (6) flexibles et abouties d'une seule pièce pour la liaison du circuit avec des composants (7) externes.

6. Circuit selon l'une quelconque des revendications 1 à 5, **caractérisé en ce que** le substrat (1) est doté de surfaces de raccordement de test (9) standardisées, qui peuvent être enlevées avant le repliage du substrat (1) équipé.

7. Circuit selon la revendication 6, **caractérisé en ce que** les surfaces de raccordement de test (9) sont disposées dans une rangée (8) située sur le bord (10) du substrat (1).

8. Circuit selon l'une quelconque des revendications 1 à 7, **caractérisé en ce qu'**un marquage (24) visible aux rayons X est disposé sur le substrat (1), de préférence sur une patte repliable et dépassant du bord du substrat.

9. Circuit selon l'une quelconque des revendications 1 à 8, **caractérisé en ce que** tous les composants (5) sur le côté à équiper (2) sont montés en particulier soudés ou collés, avec un procédé d'établissement de contact uniforme au cours d'une seule étape de fabrication.

10. Circuit selon l'une quelconque des revendications 1 à 9, **caractérisé en ce que** des composants (5.5) actifs sont disposés à califourchon sur des composants (5.6) passifs qui font office d'éléments de liaison entre le composant (5.3) actif respectif et le substrat (1).

11. Circuit selon l'une quelconque des revendications 1 à 10, **caractérisé en ce que** des composants externes, en particulier la bobine de télémétrie d'un pacemaker, défibrillateur ou similaire, sont insérés entre les deux parties (12, 13) équipées du substrat (1) plié.

12. Circuit selon l'une quelconque des revendications 1 à 11, **caractérisé par** une structuration lithographique des pistes conductrices (3).

13. Circuit selon l'une quelconque des revendications 1 à 12, **caractérisé par** une structuration faite au laser des pistes conductrices (3).
